# EUROPEAN PATENT SPECIFICATION

(11) **EP 1 493 411 B1**
(45) Date of publication and mention of the grant of the patent: **10.01.2007**
(21) Application number: 03015153.4
(22) Date of filing: 04.07.2003
(51) Int. Cl.: A61F 13/15, A61F 13/56

(54) **Absorbent product including a foam back sheet**
Absorbierender Artikel eine äussere Schaumstoffschicht enthaltend
Article absorbant avec une feuille arrière en mousse

(43) Date of publication of application: 05.01.2005
(73) Proprietor: SCA Hygiene Products AB, 405 03 Göteborg (SE)
(72) Inventor: Persson, Ulrika, 434 96 Kungsbacka (SE); Stridfeldt, Chatrine, 436 58 Hovas (SE); Lakso, Elisabeth, 444 46 Stenungsund (SE)
(74) Representative: Bergstrand, Mikael Gudmundsson

(56) References cited:
- EP-A- 1 113 772
- EP-A- 1 147 756
- WO-A-00/13637
- WO-A-01/17474
- GB-A- 2 187 659
- US-A- 3 888 255
- US-A- 6 028 017

## Description

### Field of the invention

The invention relates to an absorbent product such as an incontinence pad, sanitary pad or napkin, for absorbing bodily exudate, comprising a back sheet having an outer surface intended to face a wearer's garment (i.e. the outer surface is the side facing away from the user), wherein said back sheet comprises at least one layer of a polymeric foam material.

### Background to the invention:

An absorbent product in the meaning of the present invention comprises an absorbent element in the form of a generally planar pad (although sometimes arched), which may be made up of any suitable absorbent material. Suitable absorbent materials may include loosely associated absorbent hydrophilic material like cellulose fibres, e.g. wood pulp, regenerated cellulose or cotton fibres, which may be chemically or physically modified. The absorbent element may also include other natural or synthetic materials, such as foams or polymers.

The absorbent element may further comprise layers of materials which are fluid permeable or fluid impermeable, or may be of the type comprising one or more fluid distribution layers and a fluid storage layer comprising absorbent gelling materials, usually referred to as hydrogels, superabsorbent or hydrocolloids, dispersed homogeneously or non-homogeneously in a suitable carrier material, generally made of modified or unmodified natural or synthetic fibres.

Other components can be included in the absorbent element, such as reinforcing scrims or odour controlling agents, for example.

A fluid permeable cover sheet, typically referred to as a liner or top sheet, including natural fibres such as cellulose or regenerated cellulose, synthetic fibres such as polyester fibres or fibres made of other synthetic polymers, foams or films is provided on the body-facing side of the absorbent element, the sheet being provided for comfort and conformability and directing the fluid to the underlying absorbent element. The top sheet may also include other components with hydrophilic or hydrophobic properties, such as surfactants, to provide specific properties such as fluid permeability, or may be designed in a way (e.g. with apertures) such that only portions of it has such properties.

In known absorbent products of the prior art, the outermost layer, often an impermeable back sheet, generally has the function either of providing a liquid impermeable barrier or proving a surface giving increased friction with respect to the garment to avoid a displacement of the product in the garment. The back sheet may be made of woven and non-woven fabrics, films and sheets, for example a polymeric film such as polyethylene or cellophane, or of a polymeric cellular foam material.

The absorbent element of the absorbent product is typically placed between the top sheet and the back sheet and both sheets may be advantageously joined together at their periphery, for example by welding or adhesion.

In order to maintain the absorbent product in a certain location against the user's garment (normally the user's undergarment), the prior art products include typically an adhesive element, which is placed on a portion of the external garment-facing surface or layer of the absorbent product. The material of the adhesive element is generally selected from a large number of pressure-sensitive adhesives that are commercially available, including the cold, pressure-sensitive adhesives such as acrylate adhesives, generally combined with tackifiers such as polyterpenes, or rapid-setting thermoplastic adhesives (or hot melt adhesives) such as styrene and butadiene copolymers.

In most prior art products, the adhesive is covered by a peelable, protective strip of some type, which protects the adhesive during storage and handling prior to use. Such a protective strip may be made of a paper for example that has been treated so as to be readily releasable from the adhesive element, e.g. a paper strip treated with silicone.

However, both the aforementioned known adhesive-based and frictional-based systems, although often accepted by the consumer, have been shown to provide unsatisfactory assurance to the wearer that the absorbent product will remain in place. The conventional known adhesive-based system has the disadvantage that either the adhesive clings too tenaciously to the undergarment and therefore becomes deposited thereon, or it may create a bond which is so strong that it causes the absorbent product or the undergarment to tear when the absorbent product is being discarded after use. The conventional known frictional-based system has the disadvantage that it is effective when forces are exerted in a direction normal to the direction in which motion is to be restrained, and therefore is greatly dependent on the positions and movements of the wearer.

To reduce these problems, a solution has been suggested to combine both the adhesive-based and frictional-based systems. For example, US 3,888,255 discloses a sanitary napkin including a garment-contacting surface provided with a layer of material thereon having a high coefficient of friction and to which a second layer including a pressure sensitive adhesive is applied. This clearly has the drawback of requiring a complicated manufacturing process including associating different type of materials together.

In other cases of the prior art, to improve the attachment of the product to the garment, the back sheet layer has been provided by a slip-proofing device or has been modified to increase the friction.

For example, in GB 2 187 659, a slip-proofing device is described having non-slip means in the form of foamed polymer projections disposed on the surface of a flexible air-permeable or water-proof back sheet web.

US 4,969,970 discloses a single foam layer which includes a plurality of openings extending through the layer (each opening having a diameter of 1 to 10 mm and a transverse and longitudinal arrangement pitch of 3 to 30 mm after having been stretched). The openings allow glue on the underlying impervious sheet to contact the user's undergarment. Due to the large openings, the foam layer is permeable to fluids and is thus adhered to a non-permeable back sheet. The non-permeable back sheet between the foam layer and the absorbent core prevents body fluid from passing through the core onto the undergarment.

In other respects, materials including foam which is air-permeable and water-impermeable have also been suggested for use as back sheets for absorbent products.

For example, US 4,534,769 describes a diaper including an absorbent pad affixed between two separate sheets of elastic foam made of plastic material, one of the sheets (the bodyside layer) being air-permeable and water-permeable, the other being air-permeable but water-impermeable. This permeability is achieved due to a different reticulation level or a different thickness in the individual material sheets.

Similarly, US 6,028,017 describes a composite material made of a breathable elastic foam film substrate (consisting of a natural or synthetic elastomeric polymer material) having pores formed before or during curing of the film to provide an undisturbed contiguous structure, and a non-woven water-repellent textile formed of fibres which are attached together by needle punching, flocking, use of an adhesive or by application before the film is cured. Thus, the material is breathable and avoids the passage of fluid through the core onto the garment.

Another solution to the opposing needs of providing air permeability and liquid impermeability has been suggested in WO 00/13637 which includes by a back sheet layer of a hydrophobic foam material with open cells. The foam preferably has a thickness of less than 2 mm, and most preferably less than 1.4 mm, and the pore size of the foam cells is such that the largest individual pore dimension in the foam structure is less than 65% of the thickness of the foam in the back sheet and most preferably less than 10% of the foam thickness. The nature of the rear surface of the backsheet is not stated, but it is disclosed that coatings and other hydrophobic treatments can be applied to the surface of the foam to increase its hydrophobicity. For holding the article in place, a fastening means in the form of adhesive or mechanical attachment is used.

The materials in the aforegoing products thus attempt to provide particular breathability properties for the backsheet, but fail to provide both a guarantee of breathability and good attachment to a user's garment at the same time as giving impermeability to liquids.

In particular, these products have no specific adherence property with respect to the garment and thus additional fasteners are required, which makes them inappropriate for use when such property is required.

Another preferred aspect of absorbent products of the present invention is that their thickness should be as little as possible so as to provide the desired flexibility and pliability, for reasons of comfort.

Therefore, there is a need for a product suitable for use in absorbent products, which has both advantages of being breathable and impermeable to fluids and which has good adherence characteristics with regard to the garment.

This product should also be sufficiently versatile, in order to be adapted to situations in which the amount of liquid to be prevented from permeating through the layer is either low or high.

The object of the present invention is to provide a solution to at least some of the aforementioned problems.

A further object of the present invention is to provide a back sheet material which can be manufactured in an economical manner.

A still further object of the present invention is to provide a back sheet which may be adapted to situations in which the amount of liquid to be stopped is higher than the usual amounts which can be stopped by a conventional foam layer.

One of the advantages of the present invention is that it obviates the use of adhesive for holding the product in place against the garment. Consequently there is also no need for any protective strip to cover any adhesive.

Several properties can thus be provided at the same time from the same material. In a preferred embodiment in accordance with the present invention, the back sheet including all these properties can be made from a single layer of foam material.

Another advantage is therefore that the back sheet layer, and thus the whole absorbent product, can be manufactured in a simple manner, leading to economical and ecological improvements.

A still further advantage is that by providing several properties within the same material, the thickness of the back sheet layer, and thus of the whole absorbent product, can be maintained as little as possible and provide the desired flexibility and pliability to the finished absorbent product.

However, although the invention obviates the use of adhesive, it will be clear to a skilled person that adhesive could also be added to the portion of the back sheet facing the user's undergarment, if this is desired for any reason.

Further problems which can be solved by this invention with respect to known prior art materials used for disposable absorbent products will become apparent to the reader of the following description.

### Summary of the invention:

The present invention provides a solution to at least some of the aforementioned problems by provision of an absorbent product as defined in claim 1.

Preferred embodiments of the invention are defined in the dependent claims.

As used herein, "cells" are referred to in the foam material. These are the cells (or pores) which are present in the foam material as a result of its manufacturing process. Since the foam material of at least one layer, and preferably all foam layers, has an open cell structure, this means that the cells are open with respect to each other, thus allowing the passage of gases and vapours through the foam. Depending on the size of the openings between the cells (i.e. the size of the openings joining adjacent cells), the permeability of the structure can be changed. The size of the openings between the cells is defined here in terms of diameter, since the openings are substantially oval or circular in shape; the diameter being the largest dimension across the plane of the opening joining two cells. Although some cells, in the region of the foam material which is to be gas permeable, may be joined by openings which are greater than 20µm, it is preferable that at least 50%, preferably between 60 and 100%, and even more preferably between 80 and 100% of the openings have a size equal to or less than 20µm.

Similarly, the open cavities in the outer surface of the back sheet may be defined in terms of their diameter. The diameter here refers to the largest dimension across the plane of the open cavity, measured at the outer, garment-facing surface of the foam material, in a non-stretched and flat state. It will also be apparent that not every single cavity in the back sheet needs to have a diameter which is between 50 and 400µm, although a sufficient amount must have this size. The term sufficient amount in this context is to be understood as being an amount of cells necessary for achieving the required frictional force against an undergarment. An amount more than 20%, preferably more than 40% and more preferably between 50% and 100%, even more preferably an amount between 75% and 100%, are cavities of the stated diameter which are present in order to achieve this result.

The back sheet of the absorbent product of the invention is substantially entirely made of one or more layers of polymeric cellular foam, apart from possible adhesive for joining layers and apart from any superabsorbent material which may be added. Several layers may thus constitute the backsheet, each being a layer of polymeric cellular foam, in which the properties of each layer may be different.

The back sheet may also comprise one or more foam layers where the cell or pore size (here, the maximum dimension across a cell) of the cell structure varies within the foam structure. One example of a suitable foam is a foam layer having a cell or pore gradient whereby the gradient may vary from large (e.g. up to about 400 µm diameter) at one surface, preferably the garment-facing surface, to small (e.g. less than 10µm) at the opposing surface. A cell size gradient may additionally or alternatively be provided which changes from large at one surface to small (e.g. in the middle region, or another region somewhere between the two surfaces) and then changes to large at the other surface. Other gradients or other cell size distributions are also possible. Several separate foam layers with differing gradients may also be combined and/or fixedly attached together.

Where the back sheet is made of several layers, this may be referred to as a laminated structure. The layers should be made of a material of similar type, namely polymeric cellular foam. The foam layers may be attached together, for example with adhesive, preferably an elastomeric-adhesive, applied as a sprayed coating on one or more of the layers before joining the layers together. As will be apparent to a skilled person, the adhesive should be applied in such a way that minimal disturbance is caused to the passage of gas and/or vapours through the foam structure.

The backsheet of the invention may be formed of such a size (i.e. cross-sectional area) that it only forms part of the garment-facing surface of the absorbent article, whereby the remainder of the garment facing surface can be formed by a film material or other suitable material.

By polymeric cellular foam of similar type, it will be understood that the polymeric cellular foam is of the type "opened cell structure", with cells of the same or different diameter, or with a gradient of cell size as explained above. The external surface of the polymeric cellular foam may be modified in order to achieve the correct cavity size, although if the cells themselves are large enough and numerous enough, any special modification of the aperture size can be obviated merely by leaving the cells open at the external surface of the outermost layer (i.e. no coatings closing the apertures or closure of the foam material when forming a sheet of same are used).

The polymeric cellular foam should be of the hydrophobic type in the sense that it should not attract or absorb liquids. The foam may be of reticulated type, i.e. prepared in such a way that the foam bubbles burst during manufacture, which gives the foam its permeability across the openings between the cells.

A further way in which the foam can be prepared in order to achieve a certain size of cell, is the so-called salting-out method, whereby NaCl (table salt) can be mixed with polymeric powder and the mixture can be compression moulded or extruded into thin sheets (i.e. sheets up to a maximum of about 6.4 mm thickness). The sheets can then be soaked in hot water, typically for up to 4 hours, so as to fully dissolve out the salt. The size of the cells formed depends on the particle size distribution of the salt. Particle sizes are selected to the needs of the cell size required and thus the cell size can be relatively accurately controlled, even though the cells may be somewhat irregular in shape. Finer particle sizes (below about 100µm) can be achieved by finely grinding the salt particles prior to dosing the polymer powder. An alternative to this is to finely grind the polymer powder and the salt particles together.

The thickness of the foam layer may also be used to vary certain properties of the foam layer i.e. to make it so thick that it is liquid tight but permeable to air, but it must be ensured that the cavities in the outer surface are of the required size to provide the friction-based attachment.

By varying the thickness appropriately and/or by appropriate shaping, the foam layer can be shaped so that either a basin-like cavity is formed in the absorbent product or a hump-like protrusion.

Examples of suitable foams for the back sheet are polyurethane foams of ester or ether type, polyethylene foams, cellulosic foams, natural and synthetic latex foams, silicone foams, and polyvinyl foams (polyvinylchloride, polyvinylchloride-vinylacetate copolymers and copolymers of vinyl chloride and vinylidene chloride).

The cavities can also be obtained, or modified, by a process known per se in the art and used to modify the surface of foam layers, such as for example embossing.

The technical effect achieved by the invention is that with a back sheet made of a single type of material, it is possible to combine at the same time the adhesion requirement (frictional-based attachment due to the modification of the surface of the layer intended to face the garment) and the requirement that the material is permeable to air and non-permeable to liquids. This latter effect may for example be achieved by selection of the appropriate size of the openings between the cells.

The simplest embodiment is obtained when the back sheet is made of a single layer of foam material permeable to air and non-permeable to liquids, by using for example a foam of reticulated type with cells having a size and an opening between the cells which is controlled in order to meet the permeability requirements, and to arrange (or modify) the surface of one side of this foam layer so as to achieve the cavities required in order to meet the desired friction characteristic with regard to the garment.

The back sheet material may optionally be coated on its inner side, i.e. the side which faces the absorbent core material, with absorbent gelling materials, usually referred to as hydrogels, superabsorbent or hydrocolloids. This will be preferably used in case of high amounts of liquids to be stopped.

In one embodiment of the present invention, at least part of the surface of the layer facing the absorbent core also comprises cavities. These cavities are of such a size and shape that they can be filled, or partially filled with an absorbent gelling material by coating this or depositing this on said surface. The superabsorbent gelling material can be for example selected from hydrogels, hydrocolloids or other superabsorbent materials. One example of applying such a material to the cavities in the upper surface is by using a polyacrylate-based superabsorbent material (in other words by using the most conventional form of SAP material). This can be applied to a foam layer, suitable for the invention, having a cell size of 200µm (and also with cavities in the outer surface thereof of the same size), such as produced by Caligen Foam Ltd of Broad Oak, Accrington, Lancashire UK BB5 2BS under the foam product identification code XD100AS, whereby the SAP product may be sprayed as a monomer solution on to the upper surface of the foam layer, at a surface weight which may vary between 1 to 100 g/m², before using UV radiation or an electron beam to cross-link the monomer solution. However, it is also possible to add cross-linked superabsorbent material in particulate, or other material forms, into the cavities.

It will also be apparent to a skilled person that the size of the cavities does not heed to be exactly 200µm and that somewhat smaller cavities can be used, for example down to 100µm, and that even larger cavities can be used.

The purpose of these cavities filled with an absorbent gelling material is that, when high amounts of liquids are to be stopped by the back sheet, the absorbent gelling material present in the cavities will, in contact with the liquid, swell and clog up the cavities. Even when using just one layer of foam without any additional foam layers in the back sheet, it has been shown that the superabsorbent material prevents liquids from passing through the foam layer, despite the presence of the relatively large cavities. In this way, the material of the foam back sheet is not only used as an air-permeable liquid blocker at the surface closest to the absorbent core, but is also used to provide an additional absorbency medium. Since the cells or pores contain the SAP material, the thickness of the layer is relatively unaffected even though an increase in absorbency is obtained. At the same time, the relatively large cavities allow superior breathability characteristics until the time that large amounts of fluid have been exuded and the cavities become substantially blocked. It will also be apparent that since the liquid is stopped and retained at the level of the cavities, the surface of the back sheet facing the garment remains dry.

Additionally, with the use of superabsorbent materials as described above, even with large exudate flows the foam material will still keep its permeability to air in those places where no liquid is present. Given the relatively large size of the cells when dry, the permeability is of course also relatively high. Thus the absorbent product is particularly advantageous not only from a point of view of absorbency but also in terms of its "breathability" characteristics.

A clear advantage of this system is that it avoids the typical situation in which a high amount of liquid will lead to leakage or even to damage of the back sheet. The friction-based characteristics of the surface facing the garment can thus remain substantially unaffected.

Although particular materials and dimensions of the various components are specified herein, it will be clear to the skilled person that the materials used and the width and length of the various components may be changed in order to suit the particular circumstances, as long as the required properties are maintained.

### Brief description of the drawings:

The invention will now be described in more detail with reference to the accompanying drawings in which:

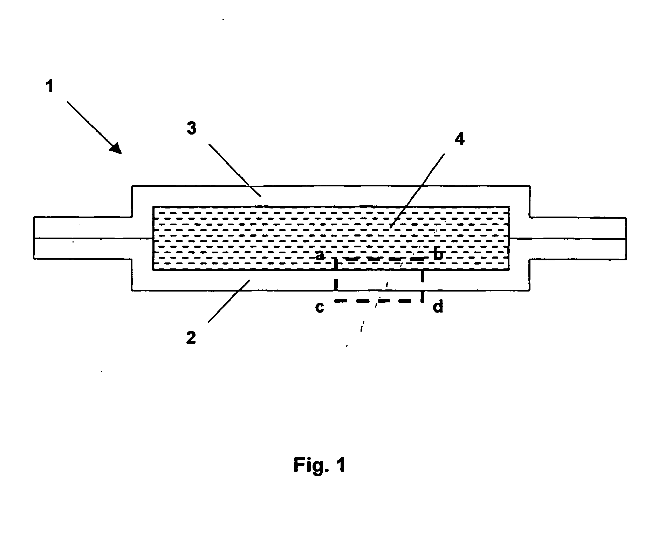

- Fig. 1: illustrates a schematic cross-sectional view of a typical disposable sanitary pad in accordance with the invention, prior to use, comprising an absorbent element placed between a top sheet and a back sheet, the back sheet surface intended for placement against the garment being shown lowermost, and the top sheet and back sheet being joined together at their periphery;

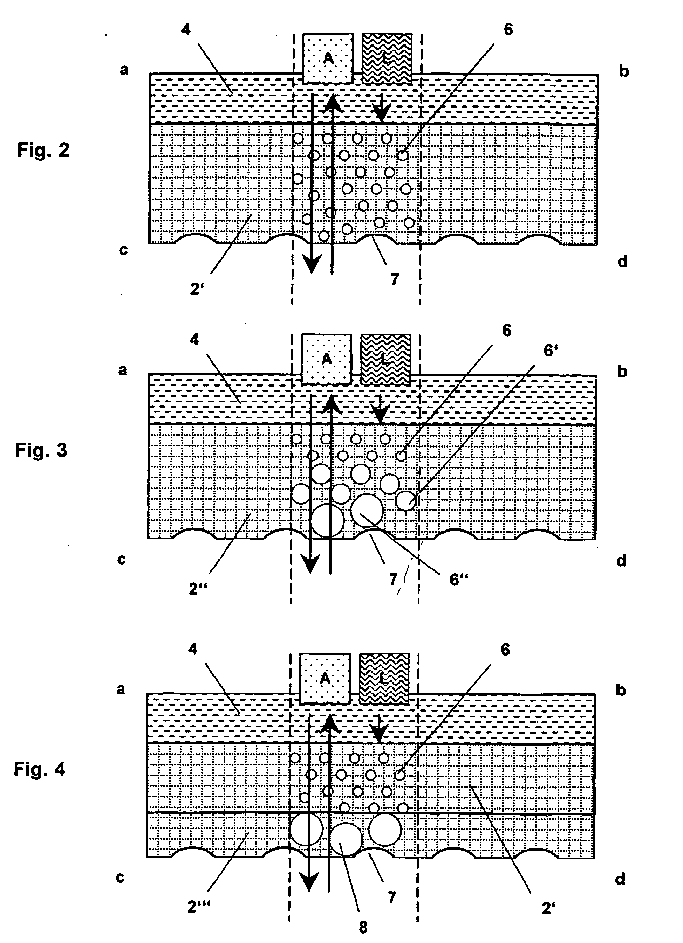

- Figs. 2-7: each illustrates a schematic plane view of the surface delimited by a-b-c-d of the sanitary pad of Fig.1, with the details of the structure of the back sheet represented schematically between the dotted lines, the passage of air in both directions through the back sheet layer being represented by two arrows and a square with an A, the stopping of the liquids at the interface of the back sheet and the absorbent element being represented by one arrow and a square with an L, and wherein:
- Figs. 2 and 5: illustrate a preferred embodiment in which the back sheet is made of a single layer of polymeric cellular foam material of the type open cell structure, the pores being of the same size;
- Figs. 3 and 6: illustrate a preferred embodiment in which the back sheet is made of a single layer of polymeric cellular foam material of the type open cell structure, the pores forming an increasing gradient of size from the side of the layer intended to face the absorbent element to the side of the layer intended to face the garment;
- Figs. 4 and 7: illustrate a preferred embodiment in which the back sheet is made of several layers of polymeric cellular foam material, the pores of each of the layers having a different size.
- Figs. 5 to 7: illustrate preferred embodiments in which at least part of the surface of the layer facing the absorbent element comprises cavities filled with an absorbent gelling material.
- Fig. 8: illustrates a testing apparatus for measuring friction of a test sample against a steel plate.
- Fig. 9: illustrates a testing apparatus for measuring friction of a test sample against a nylon or cotton material sample secured to the steel plate.

### Description of preferred embodiments:

A cross-sectional view of a typical absorbent product 1 according to the present invention and having the form of a sanitary pad is schematically shown in Fig. 1.

The absorbent product comprises an absorbent element 4 (i.e. an absorbent core) placed between a top sheet 3 and a back sheet 2, the back sheet lower surface being for placement against a wearer's garment, and the top sheet and back sheet preferably being joined together at their periphery.

The absorbent element 4 may be made up of any suitable absorbent material such as loosely associated absorbent hydrophilic material like cellulose fibres, e.g. wood pulp, regenerated cellulose or cotton fibres, which may be chemically or physically modified. The absorbent element may also include other natural or synthetic materials, such as foams or polymers. It may be made of a single or of several layers of material, and may comprise other components such as odour controlling substances or absorbent gelling materials for example.

Thus, the absorbent element 4 may be formed by any of the prior art known absorbent element types available for these kinds of absorbent products.

The top sheet 3 if formed by a liquid permeable cover sheet provided on the body-facing side of the absorbent element 4. The top sheet 3 may also have characteristics provided for comfort and conformability, and for directing the fluid to the underlying absorbent element. It may include natural fibres such as cellulose or regenerated cellulose, synthetic fibres such as polyester fibres or fibres made of other synthetic polymers, foams or films. It may also include other components or may be designed in a specific way to provide specific properties such as a control of fluid permeability. Thus, the top sheet 3 may be any top sheet available for this kind of absorbent product.

The top sheet 3 and the back sheet 2 may be joined together, preferably at their periphery, for example by welding or adhesive.

The back sheet 2, which is the most important aspect of the absorbent product of the present invention, is made of at least one layer of a polymeric foam material.

The polymeric foam material is of hydrophobic type and may preferably be of reticulated type. Suitable foam materials are the flexible compressible polymeric foams, such as the polyurethane foams of ester or ether type, polyethylene foams, cellulosic foams, natural and synthetic latex foams, silicone foams, and polyvinyl foams (polyvinylchloride, polyvinylchloride-vinylacetate copolymers and copolymers of vinyl chloride and vinylidene chloride). Polyethylene, polyurethane and cellulosic foams are however preferred.

The reticulated foam layer can generally be prepared in accordance with a process known in the art, such as the process described for example in US 6,028,017.

Generally, a thickened and whipped emulsion of the polymer is prepared to form a frothed emulsion. The frothed emulsion is cast and cured to form a cellular sheet or film. The emulsion may be whipped with a blowing agent to introduce gas to the emulsion, or the blowing agent can be a chemically generated gas such as carbon dioxide. Alternatively, the blowing agent can be air introduced mechanically to the emulsion, by frothing for example, using a mixing head. Various additives may be added with the emulsion, such as fillers, surfactants and thickeners, to enhance the processing or the resulting product. The salting-out method described previously is a further example of a method for producing such foams.

In the present invention, the terminology "pores" or "cells" will be used to describe cells as well as other apertures and/or voids in the foam layer.

Through the degree of reticulation, it is possible to choose the permeability of these foams both with respect to liquids and air.

Another process is described in WO 00/13637, which produces reticulated microporous foam structures from high internal phase emulsions, which may also be used as basic material in the present invention.

The porosity of the foam material may be determined using a porosimeter and standard procedures known in the art, such as described in US 6,028,017.

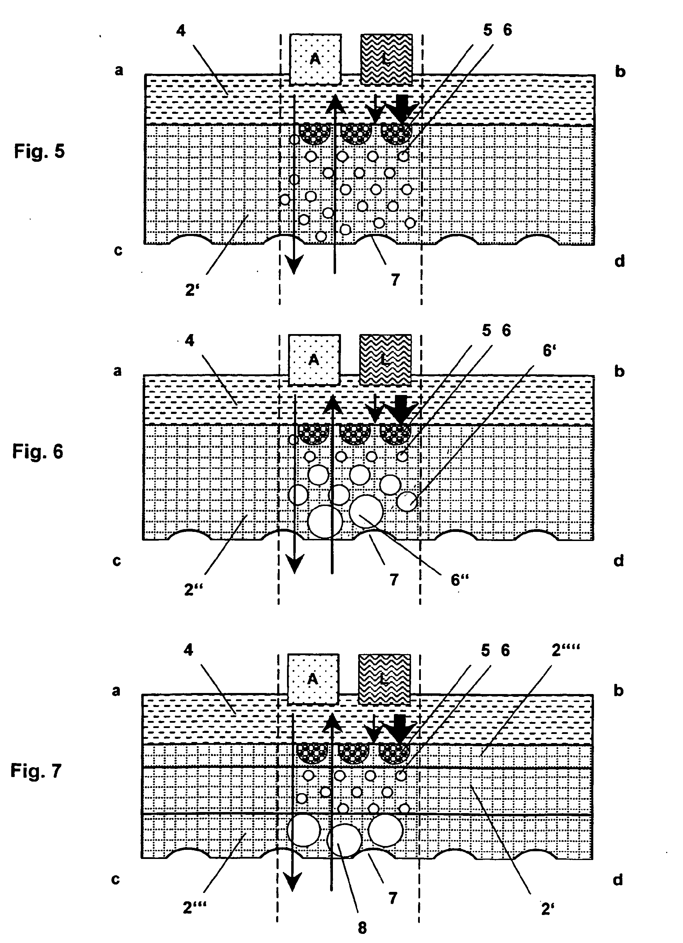

The structure of the back sheet can be a single layer structure, such as represented in Figs. 2, 3, 5, and 6, or a structure made of several layers (a laminated structure) such as represented in Figs. 4 and 7, as long as the layers are made of a material of similar type, namely polymeric cellular foam, and as long as the polymeric foam material of at least one of said layers 2', 2" has an open cell structure comprising pores 6, 6', 6"; and is permeable to air and non-permeable to liquids.

To provide a preferable structure for permeability to air and non-permeability to liquids, it has been found that the foam should be formed such that the openings between the cells or pores 6 of the layer should be of a diameter of less than 20 micrometers (µm).

The thickness of the back sheet will preferably be comprised between 1 and 5 mm, and more preferably will be less than 2 mm.

A laminated structure is obtained by attaching at least two layers of polymeric cellular foam together, preferably layers of open celled foam, such as illustrated in Figs. 4 and 7. This may be done by using an adhesive, typically by spraying the adhesive on one of the layers. Suitable adhesives are, for example, those which are rubber-based or elastomer-based. Examples of these are DISPOMELT 5000 supplied by National Strach & Chemical AB of Torsgatan 12, P.O. Box 12033, SE 600 12 Norrköping, or ECOMELT H339 UV supplied by Collano Ebnother AG of CH-6203 Sempach-station, Switzerland, each of which may be applied suitably in an amount of between 5 and 10 g/m² between adjacent layers.

The layer intended to face the garment 2', 2", 2"' comprises, on its surface, frictional-based attachment means in the form of cavities 7. These cavities 7 are preferably constituted by the cells which are left open at the outer surface of the foam layer, the cavities 7 being arranged to be of the required size. Alternatively, the cavities 7 can be obtained by a process known in the art and used to modify the surface of foam layers, such as for example embossing or otherwise forming such cavities into the surface of the layer.

To obtain the required characteristic of friction-based attachment with respect to the garment, the surface of the foam material should be modified in order that the friction characteristic, measured using the friction force test method described below, should be greater than 1 N (Newton) against steel. The frictional force should also be greater than 4N against nylon or cotton, as specified in the test below, and more preferably greater than 10 N against nylon or cotton.

### Friction force test method

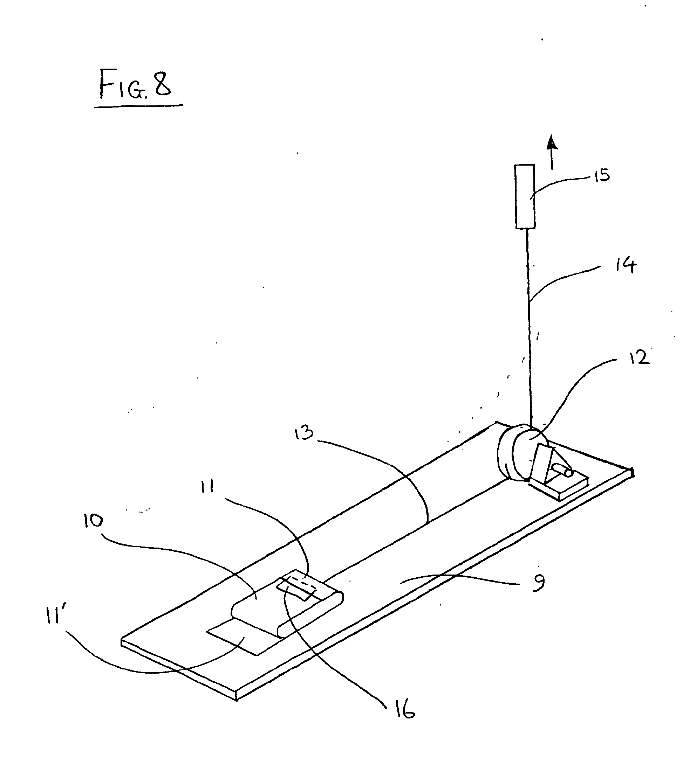

A standard test-puller is used, such as manufactured by Instron. The apparatus may also be connected to a printer. A frictional surface is provided in the form of a steel plate against which the test piece is pulled to measure friction force of the test sample against steel. The carriage has a weight of 200g ±5g. The accuracy of the measuring scale is ±0.03g. An apparatus of the type which can be used is illustrated in Fig. 8. This comprises a friction plate 9 made of steel, and a carriage 10 connected to a nylon line 13, 14 to the moving beam of a pulling device 15 on a test-puller. The pulley 12 changes the direction of force so that the line 13 is always pulled horizontally.

The test material piece 11 is a piece of back sheet 2 material cut out from the product with a size of 65 x 100 mm. The test piece should be flat and free from fingerprints and should be conditioned at 50±5% relative humidity and at 23°± 2°C for 4 hours. The test piece is fixed to the carriage 10 by fixing it with double-sided tape 16 around the curved front edge of the carriage 10 and allowing the rear end 11' of the test piece to hang free. The test puller is then zeroed.

The line 13, 14 is then passed around the pulley 12 and the line 13, 14 is tensioned to 0.05N and the test puller is zeroed again.

The pulling device 15 is subsequently set in motion and the test piece 11 is pulled at 100 mm/minute against the friction surface 9. The value obtained from the test puller is a value in Newton (N), correct to 3 decimal places and corresponds to the dynamic friction of the foam against steel.

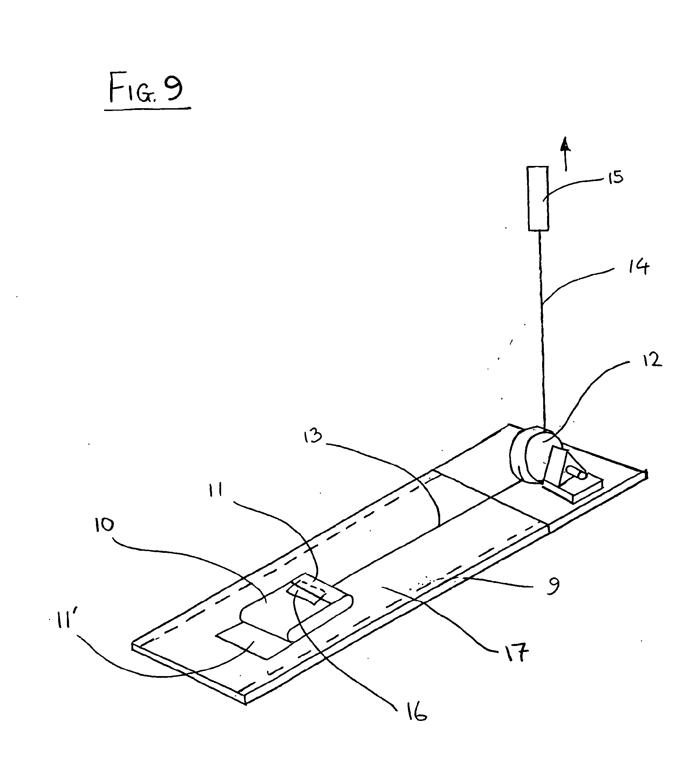

Fig. 9 illustrates the same test apparatus as in Fig. 8, with the exception that the steel plate has been covered on the majority of its upper surface with a nylon or a cotton piece 17. The nylon used for the test is of the type Cromocoll Filament Nylon 6 Tricot, Style 322 batch, Lot 7776F; the cotton used for the test being of the type known as bleached cotton interlock knit, style 460, Lot 1523. The piece 17 is stretched over and secured to the steel plate so as to provide a flat, wrinkle-free surface against which the test piece 11 can be pulled. The securement of the piece 17 to the steel plate is made on the opposite side of the plate to where the test piece 11 is positioned, the securement being by the use of double-sided tape.

In the embodiment of the present invention in which at least part of the surface of the layer facing the absorbent element 4 comprises cavities 5, illustrated in Figs. 5 to 7, the cavities are of such a size and shape that they can be filled, or partially filled with a superabsorbent material which is coated or otherwise deposited on the surface of the layer.

These cavities 5 can be obtained by laminating, on the internal side of the remainder of the back sheet, a layer 2" " of foam material having the required reticulation level to provide cavities 5 of the required size on the surface of the layer facing the absorbent element 4, such as illustrated in Fig. 7. Alternatively, a single layer 2" can be used for the backsheet, as shown in Fig. 6, in which the cavities 5 may be formed at the surface of layer 2". Thus the layer 2'' has large cavities 7 (diameter between 50µm to 400µm) at one outwardly facing surface and large cavities 5 (preferably above 100µm) at its other surface facing the absorbent core. In-between these are cells or pores 6, 6', 6"' arranged in a gradient from smaller cell sizes having reference numeral 6 to larger cell sizes having reference numeral 6".

In an alternative structure, the entire backsheet may be constituted by a single layer 2"" formed from a large cell size material (e.g. having a cell size of the order of 100 to 400µm, e.g. 200µm), with each of the outer surfaces containing respectively the open cavities 5 and 7, the cavities 5 on the surface closest to the absorbent core containing superabsorbent material in an amount sufficient to block the cavities 5 against passage of liquid when the superabsorbent material is wetted. The other arrangements of cells as disclosed herein may alternatively or additionally be incorporated into such a single layer.

Another way to obtain these cavities 5 is to prepare a foam with different reticulation levels through the thickness of the material, such as illustrated in Figs. 5 and 6.

The size of the cavities 7 should be comprised between 50 and 400µm, while the cavities 5 are preferably greater than 100µm diameter, more preferably between 100µm to 400µm.

The absorbent gelling material to be filled in the cavities 5 is, for example, superabsorbent material such as hydrogel or hydrocolloid material. These materials are well known in the art and described for example in WO 00/13637. They are typically in the form of discrete, non-fibrous particles.

The embodiments depicted in Figures 5 to 7 all show the use of superabsorbent material in cavities 5. However, the back sheets shown therein may also be used without superabsorbent material in the cavities 5, whereby at least part of the layer 2', 2", 2"' must then have a cellular structure preventing liquids finding their way to the back sheet garment-facing surface.

With the knowledge of this invention, the skilled person will therefore be able to alter the materials, width or other properties of existing products so as to produce a back sheet of absorbent product made of at least one layer of a polymeric foam material and having the required permeability and attachment characteristics described herein.

Although the present invention has been described with reference to several examples and embodiments showing specific products in specific arrangements and configurations, such is not to be considered as a limitation of the invention, but merely illustrative thereof. For example, while the invention has particular advantages with regard to incontinence pads, sanitary pads or napkins or the like, the invention may also be applied, for example, in other absorbent products where it is advantageous to hold the product in place with respect to a user's garment.

## Claims

1. Absorbent product such as an incontinence pad, sanitary pad or napkin, or other absorbent product for absorbing bodily exudate, comprising a back sheet (2) having an outer surface intended to face a wearer's garment, wherein said back sheet (2) comprises at least one layer (2', 2", 2"') of a polymeric foam material, wherein the polymeric foam material of said at least one layer (2', 2", 2"') has an open cell structure comprising cells (6, 6', 6"), and wherein at least one layer (2', 2", 2"') of said polymeric foam material is permeable to air and non-permeable to liquids, **characterised in that** the back sheet outer surface includes frictional-based attachment means in the form of open cavities (7), wherein an amount of more than 20% of said cavities (7) have a diameter measured on said outer surface of between 50 and 400 µm.

2. Absorbent product according to any one of the preceding claims, **characterized in that** said layer which is permeable to air and non-permeable to water comprises an open cell structure where the openings between the cells are less than 20µm diameter.

3. Absorbent product in accordance with claim 1 or claim 2, **characterised in that** said back sheet (2) is made of a single layer (2', 2'') of polymeric foam material.

4. Absorbent product in accordance with claim 3, **characterized in that** the material of said single layer (2', 2'') comprises said open cell structure and is permeable to air and non-permeable to liquids and, on said surface intended to face the garment, comprises said frictional-based attachment means in the form of cavities (7).

5. Absorbent product in accordance with claim 1 or 2, **characterised in that** said back sheet comprises a plurality of layers (2', 2''', 2'''') of polymeric foam material.

6. Absorbent product in accordance with any one of the preceding claims, **characterised in that** the cells (6) of the layer (2') having an open cell structure, permeable to air and non-permeable to liquids, are of equal size.

7. Absorbent product in accordance with any one of the preceding claims, **characterised in that** the cells (6, 6', 6'') of the layer (2") having an open cell structure, permeable to air and non-permeable to liquids, form an increasing gradient of size from the surface of the layer intended to face an absorbent element (4) of the absorbent product to the surface of the layer intended to face the garment.

8. Absorbent product in accordance with claim 7, **characterised in that** the cells in the foam layer furthest from the surface facing the absorbent element (4) have a size different from the size of the cells (6, 6', 6") in the polymeric foam material of the layer intended to face the absorbent element (4).

9. Absorbent product in accordance with any one of the preceding claims, **characterised in that** the cavities (7) on the surface of the foam layer (2', 2'', 2''') intended to face the garment are such that the frictional force against steel is equal to or greater than 1 N.

10. Absorbent product in accordance with any one of the preceding claims, **characterised in that** the cavities (7) on the surface of the foam layer (2', 2'', 2''') intended to face the garment are such that the frictional force against cotton or nylon is equal to or greater than 4 N.

11. Absorbent product in accordance' with claim 10, **characterised in that** the frictional force is 10 N or greater.

12. Absorbent product in accordance with any one of claims 1 to 11, **characterised in that** said back sheet (2) further comprises cavities (5) on at least part of the surface of the layer which faces away from the garment-facing surface of said backsheet, said cavities (5) containing superabsorbent material.

13. Absorbent product in accordance with claim 12, **characterised in that** said cavities (5) on at least part of the surface of the layer which faces away from the garment-facing surface of said backsheet, have a diameter greater than 100µm.

14. Absorbent product in accordance with claim 13, **characterised in that** said cavities (5) on at least part of the surface of the layer which faces away from the garment-facing surface of said backsheet, have a diameter between 100µm to 400µm.

15. Absorbent product in accordance with any one of claims 12 to 14, **characterized in that** said superabsorbent material is selected from hydrogels and hydrocolloids.

## Patentansprüche

1. Absorbierendes Produkt, wie beispielsweise ein Inkontinenzpad, eine Binde oder ein anderes absorbierendes Produkt zum Absorbieren von Körperexudaten, umfassend eine Deckschicht (2) mit einer Außenfläche, die dazu gedacht ist, zur Bekleidung des Trägers zu weisen, wobei die Deckschicht (2) wenigstens eine Lage (2', 2'', 2''') aus einem Polymerschaumstoff umfasst, wobei der Polymerschaumstoff der wenigstens einen Lage (2', 2'', 2''') eine offenzellige Struktur mit Zellen (6, 6', 6'') aufweist und wobei die wenigstens eine Lage (2', 2'', 2''') aus dem Polymerschaumstoff luftdurchlässig und flüssigkeitsundurchlässig ist, **dadurch gekennzeichnet, dass** die Außenfläche der Deckschicht eine auf Reibung basierende Befestigungseinrichtung in Form von offenen Hohlräumen (7) aufweist, wobei mehr als 20% der Hohlräume (7) einen Durchmesser gemessen auf der Außenfläche zwischen 50 und 400 µm aufweisen.

2. Absorbierendes Produkt nach Anspruch 1, **dadurch gekennzeichnet, dass** die luftdurchlässige und wasserundurchlässige Lage eine offenzellige Struktur umfasst, bei der die Öffnungen zwischen den Zellen im Durchmesser kleiner als 20 µm sind.

3. Absorbierendes Produkt nach Anspruch 1 oder 2, **dadurch gekennzeichnet, dass** die Deckschicht (2) aus einer einzigen Lage (2', 2'') aus Polymerschaumstoff gebildet ist.

4. Absorbierendes Produkt nach Anspruch 3, **dadurch gekennzeichnet, dass** das Material der einzigen Lage (2', 2'') die offenzellige Struktur umfasst und luftdurchlässig und flüssigkeitsundurchlässig ist und auf der Oberfläche, die dazu gedacht ist, der Bekleidung zugewandt zu sein, die auf Reibung basierende Befestigungseinrichtung in Form von Hohlräumen (7) umfasst.

5. Absorbierendes Produkt nach Anspruch 1 oder 2, **dadurch gekennzeichnet, dass** die Deckschicht mehrere Lagen (2', 2''', 2''''') aus Polymerschaumstoff umfasst.

6. Absorbierendes Produkt nach einem der vorstehenden Ansprüche, **dadurch gekennzeichnet, dass** die Zellen (6) der Lage (2'), die eine offenzellige Struktur aufweist und luftdurchlässig und flüssigkeitsundurchlässig ist, gleich groß sind.

7. Absorbierendes Produkt nach einem der vorstehenden Ansprüche, **dadurch gekennzeichnet, dass** die Zellen (6, 6', 6'') der Lage (2''), die eine offenzellige Struktur aufweist, luftdurchlässig und flüssigkeitsundurchlässig ist, einen zunehmenden Größengradienten von der Fläche der Lage, die dazu gedacht, einem absorbierenden Element (4) des absorbierenden Produkts zugewandt zu sein, zur Fläche der Lage, die dazu gedacht ist, der Bekleidung zugewandt zu sein, bilden.

8. Absorbierendes Produkt nach Anspruch 7, **dadurch gekennzeichnet, dass** die Zellen in der Schaumstofflage, die am weitesten von der Fläche entfernt sind, die dem absorbierenden Element (4) zugewandt ist, eine andere Größe aufweisen als die Zellen (6, 6', 6'') in dem Polymerschaumstoffmaterial der Lage, die dazu gedacht ist, dem absorbierenden Element (4) zugewandt zu sein.

9. Absorbierendes Produkt nach einem der vorstehenden Ansprüche, **dadurch gekennzeichnet, dass** die Hohlräume (7) auf der Oberfläche der Schaumstofflage (2, 2', 2'', 2'''), die dazu gedacht ist, der Bekleidung zugewandt zu sein, derart ausgebildet sind, dass die Reibungskraft bei Stahl gleich oder größer als 1 N ist.

10. Absorbierendes Produkt nach einem der vorstehenden Ansprüche, **dadurch gekennzeichnet, dass** die Hohlräume (7) auf der Oberfläche der Schaumstofflage (2, 2', 2'', 2'''), die dazu gedacht ist, der Bekleidung zugewandt zu sein, derart ausgebildet sind, dass die Reibungskraft bei Baumwolle oder Nylon gleich oder größer als 4 N ist.

11. Absorbierendes Produkt nach Anspruch 10, **dadurch gekennzeichnet, dass** die Reibungskraft 10 N oder mehr beträgt. ,

12. Absorbierendes Produkt nach einem der Ansprüche 1 bis 11, **dadurch gekennzeichnet, dass** die Deckschicht (2) ferner Hohlräume (5) auf wenigstens einem Teil der Oberfläche der Lage umfasst, die von der der Bekleidung zugewandten Fläche der Deckschicht weg weist, wobei die Hohlräume (5) ein superabsorbierendes Material enthalten.

13. Absorbierendes Produkt nach Anspruch 12, **dadurch gekennzeichnet, dass** die Hohlräume (5) auf wenigstens einem Teil der Oberfläche der Lage, die von der der Bekleidung zugewandten Fläche der Deckschicht weg weist, einen Durchmesser größer als 100 µm aufweisen.

14. Absorbierendes Produkt nach Anspruch 13, **dadurch gekennzeichnet, dass** die Hohlräume (5) auf wenigstens einem Teil der Fläche der Lage, die von der der Bekleidung zugewandten Fläche der Deckschicht weg weist, einen Durchmesser zwischen 100 µm und 400 µm aufweisen.

15. Absorbierendes Produkt nach einem der Ansprüche 12 bis 14, **dadurch gekennzeichnet, dass** das superabsorbierende Material aus Hydrogelen und Hydrokolloiden gewählt wird.

## Revendications

1. Article absorbant tel qu'une protection contre l'incontinence, un tampon hygiénique ou une serviette hygiénique, ou un autre article absorbant destiné à absorber des liquides corporels, comprenant une feuille arrière (2) comportant une surface extérieure destinée à être orientée face à un vêtement de l'utilisateur, dans lequel ladite feuille arrière (2) comprend au moins une couche (2', 2", 2''') d'un matériau polymère du type mousse, le matériau polymère du type mousse de ladite au moins une couche (2', 2", 2"') a une structure à cellules ouvertes comportant des cellules (6, 6', 6"), et dans lequel au moins une couche (2', 2", 2''') dudit matériau polymère du type mousse est perméable à l'air et non perméable aux liquides, **caractérisé en ce que** la surface extérieure de la feuille arrière comprend des moyens de fixation basés sur le frottement se présentant sous forme de cavités ouvertes (7), plus de 20 % desdites cavités (7) ayant un diamètre, mesuré sur ladite surface extérieure, compris entre 50 et 400 µm.

2. Article absorbant selon la revendication qui précède, **caractérisé en ce que** ladite couche qui est perméable à l'air et non perméable à l'eau est dotée d'une structure à cellules ouvertes dans laquelle les ouvertures entre les cellules ont un diamètre inférieur à 20 µm.

3. Article absorbant selon la revendication 1 ou 2, **caractérisé en ce que** ladite feuille arrière (2) est constituée d'une couche unique (2', 2") de matériau polymère du type mousse.

4. Article absorbant selon la revendication 3, **caractérisé en ce que** le matériau de ladite couche unique (2', 2'') est doté de ladite structure à cellules ouvertes et est perméable à l'air et non perméable aux liquides et comprend sur ladite surface destinée à être orientée face au vêtement, ledit moyen de fixation basé sur le frottement, se présentant sous forme de cavités (7).

5. Article absorbant selon la revendication 1 ou 2, **caractérisé en ce que** ladite feuille arrière comprend une pluralité de couches (2', 2", 2"') de matériau polymère du type mousse.

6. Article absorbant selon l'une quelconque des revendications qui précèdent, **caractérisé en ce que** les cellules (6) de la couche (2') dotée d'une structure à cellules ouvertes perméable à l'air et non perméable aux liquides, sont de dimension égale.

7. Article absorbant selon l'une quelconque des revendications qui précèdent, **caractérisé en ce que** les cellules (6, 6', 6") de la couche (2") dotée d'une structure à cellules ouvertes perméable à l'air et non perméable aux liquides ont des dimensions formant un gradient croissant depuis la surface de la couche destinée à être orientée vers un élément absorbant (4) de l'article absorbant, jusqu'à la surface de la couche destinée à être orientée vers le vêtement.

8. Article absorbant selon la revendication 7, **caractérisé en ce que** les cellules de la couche de mousse les plus éloignées de la surface orientée vers l'élément absorbant (4) ont une dimension qui diffère de la dimension des cellules (6, 6', 6") du matériau polymère du type mousse de la couche destinée à être orientée vers l'élément absorbant (4).

9. Article absorbant selon l'une quelconque des revendications qui précèdent, **caractérisé en ce que** les cavités (7) situées sur la surface de la couche de mousse (2', 2", 2''') destinée à être orientée vers le vêtement sont telles que la force de frottement contre l'acier est égale ou supérieure à 1 N.

10. Article absorbant selon l'une quelconque des revendications qui précèdent, **caractérisé en ce que** les cavités (7) situées sur la surface de la couche de mousse (2', 2", 2"') destinée à être orientée vers le vêtement sont telles que la force de frottement contre le coton ou le Nylon est égale ou supérieure à 4 N.

11. Article absorbant selon la revendication 10, **caractérisé en ce que** la force de frottement est égale ou supérieure à 10 N.

12. Article absorbant selon l'une quelconque des revendications 1 à 11,
**caractérisé en ce que** ladite feuille arrière (2) comprend en outre des cavités (5) sur au moins une partie de la surface de la couche qui est située à l'écart de la surface de ladite feuille arrière qui est orientée vers le vêtement, lesdites cavités (5) contenant un superabsorbant.

13. Article absorbant selon la revendication 12, **caractérisé en ce que** lesdites cavités (5) situées sur au moins une partie de la surface de la couche située à l'écart de la surface de ladite feuille arrière orientée vers le vêtement ont un diamètre supérieur à 100 µm.

14. Article absorbant selon la revendication 13, **caractérisé en ce que** lesdites cavités (5) situées sur au moins une partie de la surface de la couche qui est située à l'écart de la surface de ladite feuille arrière orientée vers le vêtement ont un diamètre compris entre 100 µm et 400 µm.

15. Article absorbant selon l'une quelconque des revendications 12 à 14, **caractérisé en ce que** ledit matériau superabsorbant est choisi parmi les gels aqueux et les colloïdes aqueux.
